# EUROPÄISCHE PATENTANMELDUNG

(11) **EP 4 606 394 A1**
(43) Veröffentlichungstag der Anmeldung: **27.08.2025**
(21) Anmeldenummer: 24159248.4
(22) Anmeldetag: 22.02.2024
(51) Int. Cl.: A61L 2/20, A61B 50/30, B65B 55/02

(54) **PHARMAZEUTISCHES GEBINDE UND VERFAHREN**

(71) Anmelder: SKAN Deutschland GmbH, 02827 Görlitz (DE)
(72) Erfinder: HOMMES, Gregor, 47669 Wachtendonk (DE); EGGERT, Benjamin, 02763 Zittau (DE); PETER, Sebastian, 02829 Markersdorf (DE)
(74) Vertreter: Mertzlufft-Paufler, Cornelius

(57) **Zusammenfassung**

Es wird ein kontaminationssensibles, insbesondere pharmazeutisches und/oder medizinaltechnisches Gebinde und eine Verwendung dessen vorgeschlagen, mit wenigstens einem Bestandteil einer pharmazeutischen und/oder medizinaltechnischen Medikationsvorrichtung und einer Verpackung, die in einem Innenraum den wenigstens einen Bestandteil aufnimmt, wobei die Verpackung in wenigstens einem den Innenraum begrenzenden Abschnitt teildurchlässig ausgebildet ist, so dass ein Dekontaminationsfluid, insbesondere Dekontaminationsgas, durchtreten kann und Partikel zurückgehalten werden, dadurch gekennzeichnet, dass im Innenraum eine das Dekontaminationsfluid abbauende Substanz präsentiert ist .

## Beschreibung

Die Erfindung betrifft ein kontaminationssensibles, insbesondere ein pharmazeutisches und/oder ein medizinaltechnisches, Gebinde mit wenigstens einem Bestandsteil einer pharmazeutischen und/oder medizinaltechnischen Medikationsvorrichtung und einer Verpackung, die in einem Innenraum den wenigstens einen Bestandteil aufnimmt, wobei die Verpackung in wenigstens einem den Innenraum begrenzenden Abschnitt teildurchlässig ausgebildet ist, so dass ein Dekontaminationsfluid durchtreten kann und Partikel zurückgehalten werden.

Derartige Vorrichtungen werden verwendet, um Bestandteile pharmazeutischer Medikationsvorrichtungen, wie beispielsweise Injektionsfläschchen, in kontrollierte Umgebungen wie Reinräume oder Isolatoren oder Containments einzubringen. Die Gebinde müssen vor einem Einbringen in die kontrollierte Umgebung sterilisiert oder dekontaminiert werden. Problematisch bei den aus dem Stand der Technik bekannten Vorrichtungen ist, dass das in das Gebinde eingedrungene Dekontaminationsfluid länger als gewünscht in den Bestandteilen pharmazeutischer und/oder medizinaltechnischer Medikationsvorrichtungen verbleiben kann und diese Bestandteile kontaminieren kann. Da diese Bestandteile immer noch reaktiv sein können, können sich aus dem Verbleib Schädigungen ergeben.

Aufgabe der Erfindungen ist daher, pharmazeutische und/oder medizinaltechnische Gebinde zu verbessern.

Zur Lösung der Aufgabe sind erfindungsgemäß die Merkmale des Anspruchs 1 vorgesehen. Insbesondere wird somit zur Lösung der genannten Aufgabe bei einem kontaminationssensiblen, insbesondere pharmazeutischen und/oder medizintaltechnischen, Gebinde der eingangs beschriebenen Art vorgeschlagen, dass im Innenraum eine das Dekontaminationsfluid abbauende und/oder aufnehmende Substanz präsentiert ist. Das Dekontaminationsfluid kann beispielsweise ein Dekontaminationsgas sein. Beispielsweise kann der Abbau des Dekontaminationsfluid durch stoffliche Umsetzung erfolgen. Pharmazeutische Gebinde und/oder medizinaltechnische Gebinde können beispielsweise in eine Schleuse zur Überführung von einem Raum mit unkontrollierter Umgebung in einen Raum mit kontrollierter Umgebung eingebracht werden. In der Schleuse wird das Gebinde von dem Dekontaminationsfluid, beispielsweise Wasserstoffperoxid oder Ozon oder Stickstoffdioxid umspült und dringt durch einen teildurchlässigen Abschnitt des Gebindes in dessen Innenraum ein. Der teildurchlässige Abschnitt, der den Innenraum des Gebindes begrenzt, kann beispielsweise dazu eingerichtet sein, Partikel zurückzuhalten. Die Bestandteile pharmazeutischer Medikationsvorrichtungen, die sich im Innenraum des Gebindes befinden, werden beispielsweise durch das Dekontaminationsfluid kontaminiert, was bei einem späteren Arbeitsschritt in der kontrollierten Umgebung unerwünscht sein kann, da das Dekontaminationsfluid mit chemischen Substanzen in der kontrollierten Umgebung reagieren kann. Mit der das Dekontaminationsfluid abbauenden Substanz kann das Dekontaminationsfluid zuverlässig und schnell abgebaut werden, damit dieses vor Überführung des Gebindes in eine kontrollierten Umgebung, beispielsweise in einen Isolator, abgebaut und somit nicht mehr vorhanden sind.

Eine das Dekontaminationsfluid aufnehmende Substanz kann beispielsweise ein Speicher und/oder ein Adsorbens, beispielsweise ein Aktivkohlefilter (z.B. Aktivkohlepads) und/oder wenigstens ein anderes kohlenstoffhaltiges Adsorbens, wenigstens ein oxidisches Adsorbens (z. B. Aktivtonerden, Kieselgele, Zeolithe, Trass), wenigstens ein Polymeradsorbens (z.B. Stryrolpolymere), Mischsorbentien (z. B. Mischung aus Kalkhydrat und Aktivkoks), metallorganische Gerüstverbindungen, und/oder allgemein Materialien mit erhöhter Porösität sein. Diese Materialien können auch miteinander kombiniert werden.

Abbauende und aufnehmende Substanzen können auch miteinander kombiniert werden.

Bei einer vorteilhaften Ausgestaltung kann vorgesehen sein, dass die Substanz in einem begrenzten Bereich des Innenraums bereitgehalten wird. Hierdurch kann beispielsweise erreichbar sein, dass die Substanz nicht den ganzen Innenraum des Gebindes befüllen muss. Von der Substanz muss nur die Menge oder das Volumen vorhanden sein, um das Dekontaminationsfluid effektiv und effizient abzubauen.

Bei einer vorteilhaften Ausgestaltung kann vorgesehen sein, dass der Bereich vom übrigen Innenraum durch eine gasdurchlässige Oberfläche zugänglich ist. Hierdurch kann beispielsweise erreichbar sein, dass die Substanz nicht in direkten Kontakt mit den Bestandteilen pharmazeutischer Gebinde kommt und diese verunreinigt. Vielmehr genügt es, wenn die Substanz durch eine gasdurchlässige Oberfläche, beispielsweise eines im Gebinde abgetrennten Raumes, zugänglich ist. Alternativ oder zusätzlich kann vorgesehen sein, dass der Bereich vom übrigen Innenraum durch eine poröse Oberfläche zugänglich ist. Hierdurch kann beispielsweise erreichbar sein, dass die Substanz hinter einer porösen Oberfläche angeordnet ist und durch diese dem Innenraum des Gebindes zugänglich ist. Alternativ oder zusätzlich kann vorgesehen sein, dass die das Dekontaminationsfluid abbauende Substanz zwischen dem Abschnitt und der Oberfläche angeordnet ist. Hierbei kann der Abschnitt als, vorzugsweise poröse und/oder gasdurchlässige, Schutzschicht ausgebildet sein.

Hierdurch kann beispielsweise erreichbar sein, dass das Dekontaminationsfluid zuerst den Abschnitt, der als Schutzschicht ausgebildet sein kann, durchdringen muss und einen Teil des Dekontaminationsfluids zurückhalten kann, bevor es durch die gasdurchlässige und/oder poröse Oberfläche in den Innenraum gelangen kann. Wird die das Dekontaminationsfluid abbauende Substanz zwischen dem Abschnitt und der gasdurchlässigen und/oder porösen Oberfläche angeordnet, kann beispielsweise das Dekontaminationsfluid vorteilhaft abgebaut werden, bevor es die Bestandteile erreicht. Es kann vorgesehen sein, dass der Abschnitt, die Substanz und die Oberfläche später in einem Arbeitsschritt gemeinsam von dem Gebinde entfernt werden können. Die Schutzschicht kann beispielsweise ebenfalls porös ausgebildet sein oder poröse Bereiche aufweisen. Die Schutzschicht kann weniger durchlässig oder durchlässiger oder genauso durchlässig sein wie der Abschnitt der Verpackung.

Allgemein kann somit die Verpackung doppelwandig oder mehrlagig ausgebildet sein. Hierbei kann der Abschnitt die äußerste Schicht bilden.

Bei einer vorteilhaften Ausgestaltung kann vorgesehen sein, dass der Bereich mit einem gasdurchlässigen Träger versehen ist. Hierdurch kann beispielsweise erreichbar sein, dass das Dekontaminationsfluid durch den Träger strömen kann. Alternativ oder zusätzlich kann vorgesehen sein, dass der Träger porös ist. Hierdurch kann beispielsweise erreichbar sein, dass Dekontaminationsfluid in die Poren der Substanz eindringen kann. Insbesondere kann vorgesehen sein, dass der Träger ein Festkörper ist. Hierdurch kann beispielsweise erreichbar sein, dass der Festkörper das Dekontaminationsfluid besonders gut abbauen kann. Insbesondere ist vorgesehen, dass der Träger den Bereich ausfüllt. Hierdurch kann beispielsweise erreichbar sein, dass genügend Substanz zum Abbau des Dekontaminationsfluides zur Verfügung steht und/oder dass die Substanz im Bereich gleichmäßig verteilt ist.

Bei einer vorteilhaften Ausgestaltung kann vorgesehen sein, dass der Bereich in einem Transportweg, insbesondere Diffusionsweg und/oder Konvektionsweg und/oder Strömungsweg, zwischen dem Abschnitt und dem Bestandteil angeordnet ist. Hierdurch kann beispielsweise erreichbar sein, dass das Dekontaminationsfluid abgebaut wird bevor es das Bestandteil erreicht. Die Kontamination des Bestandteils durch das Dekontaminationsfluid kann somit verhindert werden. Alternativ oder zusätzlich kann vorgesehen sein, dass der Bereich in einem Diffusionsweg zwischen dem Abschnitt und dem Bestandteil ausgebildet ist.

Hierdurch kann beispielsweise erreichbar sein, dass der Bereich mit der Substanz Teil des Gebindes, insbesondere beispielsweise von dessen Verpackung, ist.

Bei einer vorteilhaften Ausgestaltung kann vorgesehen sein, dass die Substanz ein vorzugsweise metallischer Katalysator ist. Hierdurch kann beispielsweise ein besonders effektiver und effizienter Abbau des Dekontaminationsfluides erreichbar sein.

Bei einer vorteilhaften Ausgestaltung kann vorgesehen sein, dass der Abschnitt mit einem Vliesstoff gebildet ist. Hierdurch kann erreichbar sein, dass der Abschnitt gasdurchlässig ist und Partikel zurückhält. Partikel können mikro- oder makroskopische Festkörper sein oder auch unerwünschte Fluide in flüssiger oder gasförmiger Form. Insbesondere kann der Vliesstoff aus Polyethylen gebildet sein. Hierdurch kann beispielsweise erreichbar sein, dass der Vliesstoff besonders strapazierfähig und flexibel ist. Alternativ oder zusätzlich kann vorgesehen sein, dass der Vliesstoff aus Fasern hoher Dichte gebildet ist. Hierdurch kann beispielsweise erreichbar sein, dass der Vliesstoff besonders dicht und undurchlässig für unerwünschte Stoffe ist. Alternativ oder zusätzlich kann vorgesehen sein, dass der Vliesstoff aus fibrillierten Feinstfilamenten gebildet ist. Hierdurch kann beispielsweise erreichbar sein, dass der Vliesstoff einerseits wasserabweisend und andererseits gasdurchlässig ist. Alternativ oder zusätzlich kann vorgesehen sein, dass der Vliesstoff aus eng miteinander zu Netzwerken verbundenen Feinstfilamenten gebildet ist. Hierdurch kann der Vliesstoff eine hohe Reißfestigkeit und Steifigkeit aufweisen.

Bei einer vorteilhaften Ausgestaltung kann vorgesehen sein, dass der Bereich durch eine im Innenraum liegende gebildete Abdeckung der Bestandteile definiert ist. Hierdurch kann beispielsweise erreichbar sein, dass die Bestandteile durch die Abdeckung von dem Bereich getrennt sind. Vorzugsweise kann vorgesehen sein, dass der Bereich durch eine im Innenraum liegende, als textiles Flächengebilde gebildete, Abdeckung der Bestandteile definiert ist. Hierdurch kann beispielsweise eine Trennung von dem Bereich des Innenraums zum restlichen Innenraum durch ein textiles Flächengebilde erreichbar sein. Insbesondere ist das textile Flächengebilde aus Vliesstoff. Hierdurch kann beispielsweise erreichbar sein, dass der Bereich durch ein im Innenraum aus Vliesstoff gebildete Abdeckung der Bestandteile definiert ist. So können die vorteilhaften Materialeigenschaften von Vliesstoff, wie beispielsweise Gasdurchlässigkeit, Partikelundurchlässigkeit, geringes Gewicht, hohe Verformbarkeit, hohe Strapazierfähigkeit und geringer Faserverlust ausgenutzt werden.

Bei einer vorteilhaften Ausgestaltung kann vorgesehen sein, dass der Katalysator als Beschichtung auf einen Träger aufgetragen ist. Hierdurch kann beispielsweise erreichbar sein, dass der Träger den Katalysator präsentiert und der Katalysator den Abbau des Dekontaminationsfluides bewirkt. Insbesondere kann vorgesehen sein, dass der Katalysator als Beschichtung auf einen porösen Träger aufgetragen ist. Hierdurch kann beispielsweise erreichbar sein, dass Dekontaminationsfluid, durch eine vergrößerte Oberfläche des Katalysators, besonders schnell abgebaut werden kann.

Alternativ oder zusätzlich sind zur Lösung der genannten Aufgabe und/oder als eine erfindungsgemäße Anwendung der Erfindung eine Verwendung einer ein Dekontaminationsfluid abbauenden und/oder aufnehmenden (siehe bspw. oben) Substanz in einem Innenraum einer Verpackung eines kontaminationssensiblen pharmazeutischen und/oder medizinaltechnischen Gebindes vorgesehen, wobei in dem Innenraum wenigstens ein Bestandteil einer pharmazeutischen und/oder medizinaltechnischen Medikationsvorrichtung aufgenommen ist und die Verpackung in wenigstens einem den Innenraum begrenzenden Abschnitt teildurchlässig ausgebildet ist, so dass ein Dekontaminationsfluid, insbesondere Dekontaminationsgas, durchtreten kann und Partikel zurückgehalten werden. Hierdurch kann beispielsweise erreichbar sein, dass das Dekontaminationsfluid in den Innenraum der Verpackung eindringen kann und dort von der Substanz abgebaut wird. Der teildurchlässige Abschnitt verhindert dabei ein Eindringen unerwünschter Partikel in den Innenraum. Der Bestandteil der pharmazeutischen Medikationsvorrichtung kann beispielsweise ein Injektionsfläschchen sein.

Eine bevorzugte Anwendung der Erfindung sieht vor, dass die Substanz auf einem Diffusionspfad zwischen dem teildurchlässigen Abschnitt und dem wenigstens einen Bestandteil präsentiert wird. Hierdurch kann beispielweise erreichbar sein, dass das Dekontaminationsfluid durch die Substanz abgebaut wird, bevor sie den Bestandteil erreicht.

Somit wird eine Kontamination des Bestandteils mit dem Dekontaminationsfluid verhindert werden. Alternativ oder zusätzlich kann vorgesehen sein, dass die das Dekontaminationsfluid abbauende Substanz zwischen einer Oberfläche und einer, die Oberfläche nach außen begrenzenden, Schutzschicht präsentiert wird. Hierdurch kann beispielsweise erreichbar sein, dass das Dekontaminationsfluid zuerst eine Schutzschicht durchdringen muss, die einen Teil des Dekontaminationsfluids zurückhalten kann, bevor es durch die gasdurchlässige und/oder poröse Oberfläche in den Innenraum gelangen kann.

Eine bevorzugte Anwendung der Erfindung sieht vor, dass die Substanz auf einem gasdurchlässigen Träger präsentiert wird. Hierdurch kann beispielsweise erreichbar sein, dass das Dekontaminationsfluid den Träger durchströmen kann und dabei abgebaut werden kann. Alternativ oder zusätzlich kann vorgesehen sein, dass die Substanz auf einem porösen Träger präsentiert wird. Der poröse Träger kann eine vergrößerte Oberfläche bereitstellen wodurch das Dekontaminationsfluid besonders schnell abgebaut werden kann. Insbesondere kann der Träger ein Festkörper sein. Hierdurch kann beispielsweise erreichbar sein, dass er eine poröse und/oder gasdurchlässige Struktur haben kann. Alternativ oder zusätzlich kann vorgesehen sein, dass der Träger ein textiles Flächengebilde ist. Hierdurch kann beispielsweise erreichbar sein, dass der Träger ein besonders geringes Gewicht aufweist und eine hohe Porosität und Permeabilität aufweist.

Eine bevorzugte Anwendung der Erfindung sieht vor, dass das Dekontaminationsfluid außerhalb des Innenraums ausgebracht wird. Hierdurch kann beispielsweise erreichbar sein, dass die Verpackung von außen dekontaminiert wird.

Eine bevorzugte Anwendung der Erfindung sieht vor, dass das pharmazeutische Gebinde nach den zuvor beschriebenen Ausgestaltungen ausgebildet ist.

Zur Lösung der eingangs genannten Aufgabe ist alternativ oder zusätzlich erfindungsgemäß eine Dekontaminationsvorrichtung mit Mitteln zur Ausführung einer der zuvor beschriebenen Verwendungen vorgesehen. Hierdurch können die zuvor beschriebenen Vorteile erreichbar sein. Insbesondere kann vorgesehen sein, dass die Dekontaminationsvorrichtung mit Mitteln zur Ausführung einer oder der zuvor beschriebenen Verwendung/en innerhalb einer Schleuse für pharmazeutische Gebinde, die in eine kontrollierte Umgebung führt, angeordnet ist. Hierdurch können die zuvor beschriebenen Vorteile innerhalb einer Schleuse für pharmazeutische Gebinde, die in eine kontrollierte Umgebung führt, erreichbar sein. Die Dekontaminationsvorrichtung kann eine Dekontaminationsquelle zum Freisetzen des Dekontaminationsfluides umfassen. Hierdurch kann beispielweise erreichbar sein, dass Oberflächen von dem Gas umströmt werden und dabei dekontaminiert werden. Alternativ oder zusätzlich kann die Dekontaminationsvorrichtung auch eine Dekontaminationsquelle zum Freisetzen eines Elektronenstrahls zur Elektronenstrahl-Sterilisation (E-Beam) umfassen. Hierdurch können effektiv chemische Verbindungen, DNA und/oder die Vermehrungsfähigkeit von Mikroorganismen zerstört werden. Alternativ oder zusätzlich kann die Dekontaminationsvorrichtung auch eine Dekontaminationsquelle zur Aussendung von UV-Strahlen umfassen. Hierdurch kann beispielweise erreichbar sein, dass die UV-Strahlen Mikroorganismen zerstören oder inaktivieren können.

Die Erfindung wird nun anhand von Ausführungsbeispielen näher beschrieben, ist jedoch nicht auf die Ausführungsbeispiele beschränkt. Weitere Ausführungsbeispiele ergeben sich durch Kombination der Merkmale einzelner oder mehrerer Ansprüche untereinander und/oder mit einzelnen oder mehreren Merkmalen der Ausführungsbeispiele.

Es zeigen
- Fig. 1: ein pharmazeutisches Gebinde in einer Schleuse die an eine kontrollierte Umgebung angeschlossen ist,
- Fig. 2: ein pharmazeutisches Gebinde,
- Fig. 3: ein weiteres pharmazeutisches Gebinde,
- Fig. 4: ein weiteres pharmazeutisches Gebinde,
- Fig. 5: ein weiteres pharmazeutisches Gebinde,
- Fig. 6: ein weiteres pharmazeutisches Gebinde und
- Fig. 7: ein weiteres pharmazeutisches Gebinde.

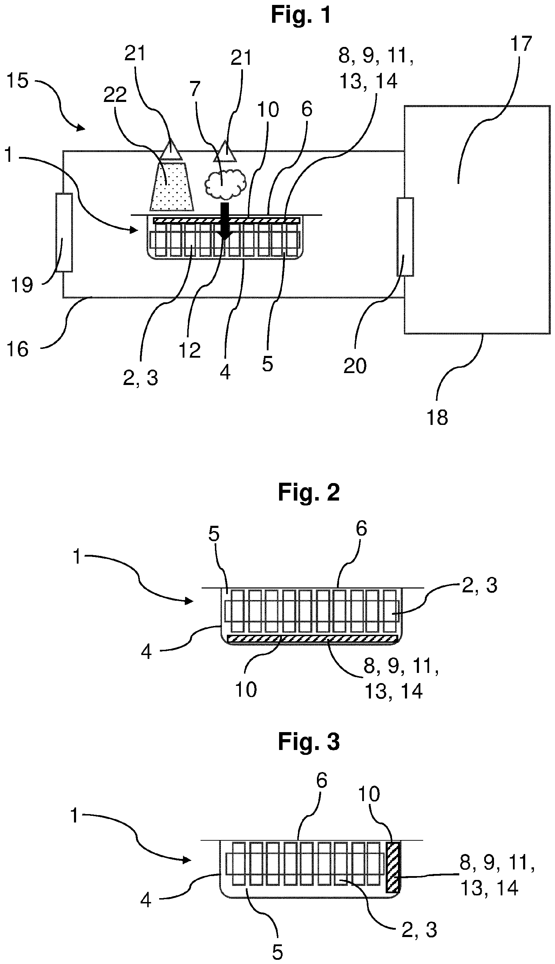

Fig. 1 zeigt ein pharmazeutisches/medizinaltechnisches Gebinde 1 mit Bestandteilen 2 einer pharmazeutischer/medizinaltechnischen Medikationsvorrichtung 3 und einer Verpackung 4. Die pharmazeutische Medikationsvorrichtung 3 sind in den gezeigten Beispielen längliche Fläschchen. Die Verpackung 4 beinhaltet die Bestandteile 2 in einem Innenraum 5. Der Verpackung 4 ist in einem den Innenraum 5 begrenzenden Abschnitt 6 teildurchlässig ausgebildet. Der Abschnitt 6 ist in den gezeigten Ausführungsbeispielen eine Abdeckung des Gebindes 1. Die Abdeckung ist als Tyvek-Vliesstoff ausgeführt und verschließt das Gebinde 1. Ein Dekontaminationsfluid 7 kann durch den teildurchlässigen Abschnitt 6 durchtreten, jedoch hält der Abschnitt 6 Partikel zurück. Die Partikel 6 können unerwünschte Verunreinigungen wie Staub aber auch größere Gasmoleküle sein. Im Innenraum 6 ist eine das Dekontaminationsfluid 7 abbauende und/oder aufnehmende Substanz 8 präsentiert. In Fig. 1 ist die Substanz 8 oberhalb der Bestandteile 2 angeordnet, so dass diese von der Substanz 8 bedeckt werden.

Die Substanz 8 wird in einem begrenzten Bereich 9 des Innenraums 5 bereitgehalten. Der Bereich 9 ist vom übrigen Innenraum 5 durch eine gasdurchlässige Oberfläche 10 zugänglich. In einem nicht gezeigten Ausführungsbeispiel ist der Bereich 9 vom übrigen Innenraum durch eine poröse Oberfläche 10 zugänglich. Der Bereich 9 ist Teil des Innenraums 5 der Verpackung 4. Der Bereich 9 ist mit einem gasdurchlässigen Träger 11 versehen. In den gezeigten Ausführungsbeispielen ist der Bereich 9 mit dem gasdurchlässigen Träger 11 ausgefüllt. In einem nicht gezeigten Ausführungsbeispiel ist die Substanz 8 zwischen dem Abschnitt 6 und der Oberfläche 10 angeordnet. Der Abschnitt 6 ist dabei als poröse und teilweise gasdurchlässige Schutzschicht ausgebildet.

In dem Ausführungsbeispiel in Fig. 1 ist gezeigt, dass der Bereich 9 ist in einem Diffusionsweg 12 zwischen dem Abschnitt 6 und dem Bestandteil 2 angeordnet ist. Das Dekontaminationsfluid 7 tritt durch den teildurchlässigen Abschnitt 6 hindurch und trifft zunächst auf den gasdurchlässigen Träger 11 auf dem die Substanz 8 präsentiert wird. Da der Träger 11 im Diffusionsweg 12 den Bestandteilen 2 vorgelagert geschieht, kann das Dekontaminationsfluid 7 abgebaut werden, bevor es die Bestandteile 2 erreicht. Die Substanz 8 ist ein metallischer Katalysator 13.

Der Abschnitt 6 ist mit einem Vliesstoff 14 aus Polyethylen gebildet. In nicht gezeigten Ausführungsbeispielen ist der Abschnitt 6 aus Vliesstoff 14 aus fasern hoher Dichte und fibrillierten und eng miteinander zu Netzwerken verbundenen Feinstfilamenten gebildet.

Der Bereich 9 ist durch eine im Innenraum 5 liegende, als textiles Flächengebilde aus Vliesstoff gebildete, Abdeckung der Bestandteile 2 definiert. Zum Abbau des Dekontaminationsfluides 7 ist der Katalysator 13 als Beschichtung auf einen porösen Träger 11 aufgetragen.

Die Substanz 8 wird dazu verwendet, das Dekontaminationsfluid 7 abzubauen. Hierzu wird sie in dem Innenraum 5 der Verpackung 4 des pharmazeutischen Gebindes 1 vorgehalten. Im Innenraum 5 befindet sich ebenfalls ein Bestandteil 2 der pharmazeutischen Medikationsvorrichtung 3. Die Verpackung 4 hat den den Innenraum 5 begrenzenden Abschnitt 6, der teildurchlässig ausgebildet ist, so dass das Dekontaminationsfluid 7 durchtreten kann und Partikel zurückgehalten werden.

Die Substanz 8 wird auf einem Diffusionsweg 12 zwischen dem teildurchlässigen Abschnitt 6 und dem Bestandteil 2 präsentiert. Die Substanz wird auf einem gasdurchlässigen und porösen Festkörper, der ein textiles Flächengebilde ist, präsentiert. Das Dekontaminationsfluid 7 wird zur Dekontaminierung der Verpackung 4 außerhalb des Innenraums 5 ausgebracht. In einem nicht gezeigten Ausführungsbeispiel verläuft der Diffusionsweg 12 in den Innenraum 5 des Gebindes 1 von außen durch eine Schutzschicht und eine gasdurchlässige und/oder poröse Oberfläche 10, wobei zwischen der Schutzschicht und der Oberfläche, die das Dekontaminationsfluid abbauenden Substanz präsentiert ist.

Fig. 1 zeigt eine Dekontaminationsvorrichtungen 15 zum Dekontaminieren einer Verpackung 4 eines pharmazeutischen Gebindes 1 innerhalb einer Schleuse 16 für pharmazeutische Gebinde 1, die in eine kontrollierte Umgebung 17 führt. Die kontrollierte Umgebung ist im gezeigten Ausführungsbeispiel ein Isolator 18. Die Dekontaminationsvorrichtung 15 umfasst im gezeigten Ausführungsbeispiel zwei Dekontaminationsquellen 21. Eine Elektronenstrahl-Sterilisation (E-Beam) 22 und das Dekontaminationsfluid 7. Die beiden Dekontaminationsquellen 21 können gemeinsam oder auch nur einzeln für sich an der Schleuse 16 ausgebildet sein. Das Gebinde 1 wird durch einen Port 19 der Schleuse 16 in die Schleuse 16 eingebracht und nach der Dekontaminierung durch einen Port 20 des Isolator 18 in die kontrollierte Umgebung 17 des Isolators 18 überführt.

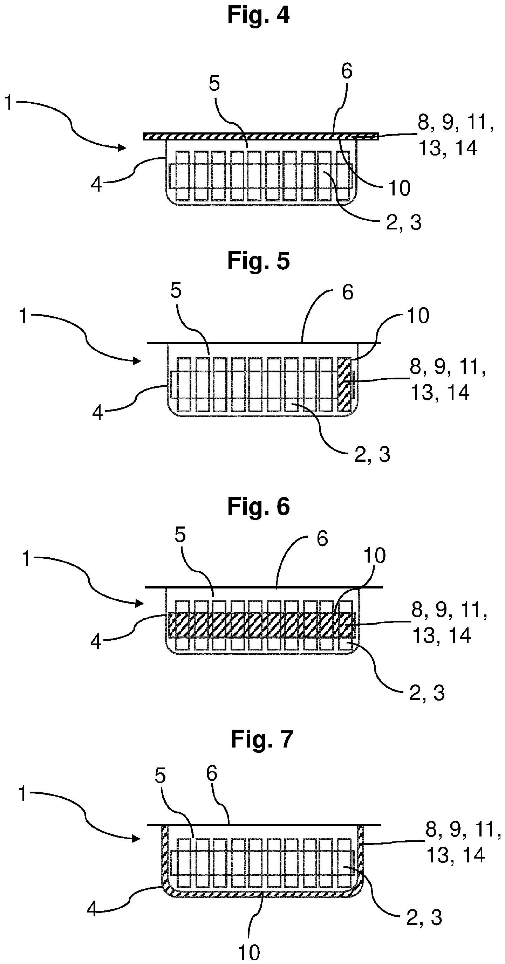

Fig. 2 zeigt ein Ausführungsbeispiel, bei dem die Substanz 8 unterhalb der Bestandteile 2 angeordnet ist. In Fig. 3 befindet sich die Substanz 8 an einem Rand des Gebindes 1. In Fig. 4 ist gezeigt, dass die Substanz 8 auch als der Bereich 9 ausgebildet sein, kann der den Innenraum 5 des Gebindes 1 von einem Außenraum gasdurchlässig abtrennt. Fig. 5 zeigt beispielhaft, dass die Substanz 8 auch als ein oder in einem Bestandteil 2 des Gebindes 1 vorgehalten werden kann. Die Substanz 8 kann die Bestandteile 2 auch, wie in Fig. 6 gezeigt, auf einer mittleren Höhe des Gebindes umgeben. Ebenfalls kann die Substanz 8 auch den Innenraum 5 der Verpackung 4 des Gebindes 1 auskleiden, wie in Fig. 7 gezeigt.

Es wird ein kontaminationssensibles, insbesondere pharmazeutisches und/oder medizinaltechnisches Gebinde 1 und eine Verwendung dessen vorgeschlagen, mit wenigstens einem Bestandteil 2 einer pharmazeutischen und/oder medizinaltechnischen Medikationsvorrichtung 3 und einer Verpackung 4, die in einem Innenraum 5 den wenigstens einen Bestandteil 2 aufnimmt, wobei die Verpackung 4 in wenigstens einem den Innenraum 5 begrenzenden Abschnitt 6 teildurchlässig ausgebildet ist, so dass ein Dekontaminationsfluid 7, insbesondere Dekontaminationsgas, durchtreten kann und Partikel zurückgehalten werden, dadurch gekennzeichnet, dass im Innenraum 5 eine das Dekontaminationsfluid 7 abbauende Substanz 8 präsentiert ist.

### Bezugszeichenliste

- 1: Gebinde
- 2: Bestandteile
- 3: Medikationsvorrichtung
- 4: Verpackung
- 5: Innenraum
- 6: Abschnitt
- 7: Dekontaminationsfluid
- 8: Substanz
- 9: Bereich
- 10: Oberfläche
- 11: Träger
- 12: Diffusionsweg
- 13: Katalysator
- 14: Vliesstoff
- 15: Dekontaminationsvorrichtung
- 16: Schleuse
- 17: kontrollierte Umgebung
- 18: Isolator
- 19: Port der Schleuse
- 20: Port des Isolators
- 21: Dekontaminationsquelle
- 22: E-Beam

## Patentansprüche

1. Kontaminationssensibles, insbesondere pharmazeutisches und/oder medizinaltechnisches, Gebinde (1), mit wenigstens einem Bestandteil (2) einer pharmazeutischen und/oder medizinaltechnischen Medikationsvorrichtung (3) und einer Verpackung (4), die in einem Innenraum (5) den wenigstens einen Bestandteil (2) aufnimmt, wobei die Verpackung (4) in wenigstens einem den Innenraum (5) begrenzenden Abschnitt (6) teildurchlässig ausgebildet ist, so dass ein Dekontaminationsfluid (7), insbesondere Dekontaminationsgas, durchtreten kann und Partikel zurückgehalten werden, **dadurch gekennzeichnet, dass** im Innenraum (5) eine das Dekontaminationsfluid (7) abbauende und/oder aufnehmende Substanz präsentiert ist.

2. Kontaminationssensibles Gebinde (1) nach dem vorangehenden Anspruch, **dadurch gekennzeichnet, dass** die Substanz (8) in einem begrenzten Bereich (9) des Innenraums (5) bereitgehalten wird.

3. Kontaminationssensibles Gebinde (1) nach dem vorangehenden Anspruch, **dadurch gekennzeichnet, dass** der Bereich (9) vom übrigen Innenraum (5) durch eine gasdurchlässige und/oder poröse Oberfläche (10) zugänglich ist und /oder dass die Substanz (8) in dem Bereich (9) zwischen dem Abschnitt (6) und der Oberfläche (10) angeordnet ist.

4. Kontaminationssensibles Gebinde (1) nach dem vorangehenden Anspruch, **dadurch gekennzeichnet, dass** der Bereich mit einem gasdurchlässigen und/oder porösen Träger (11), insbesondere Festkörper, versehen, insbesondere ausgefüllt, ist.

5. Kontaminationssensibles Gebinde (1) nach dem vorangehenden Anspruch, **dadurch gekennzeichnet, dass** der Bereich (9) in einem Diffusionsweg (12) zwischen dem Abschnitt (6) und dem Bestandteil (2) angeordnet und/oder ausgebildet ist.

6. Kontaminationssensibles Gebinde (1) nach einem der vorangehenden Ansprüche, **dadurch gekennzeichnet, dass** die Substanz (8) ein vorzugsweise metallischer Katalysator (13) ist.

7. Kontaminationssensibles Gebinde (1) nach dem vorangehenden Anspruch, **dadurch gekennzeichnet, dass** der Abschnitt (6) mit einem Vliesstoff (14), insbesondere aus Polyethylen und/oder aus Fasern hoher Dichte und/oder aus fibrillierten und/oder eng miteinander zu Netzwerken verbundenen Feinstfilamenten, gebildet ist.

8. Kontaminationssensibles Gebinde (1) nach dem vorangehenden Anspruch, **dadurch gekennzeichnet, dass** der Bereich (9) durch eine im Innenraum (5) liegende, vorzugsweise als textiles Flächengebilde, insbesondere aus Vliesstoff (14), gebildete, Abdeckung der Bestandteile (2) definiert ist.

9. Kontaminationssensibles Gebinde (1) nach dem vorangehenden Anspruch, **dadurch gekennzeichnet, dass** der Katalysator (13) als Beschichtung auf einen, vorzugsweise porösen, Träger (11) aufgetragen ist.

10. Verwendung einer ein Dekontaminationsfluid (7), insbesondere Dekontaminationsgas, abbauenden und/oder aufnehmenden Substanz (8) in einem Innenraum (5) einer Verpackung (4) eines kontaminationssensibles, insbesondere pharmazeutischen und/oder medizinaltechnischen, Gebindes (1), wobei in einem Innenraum (5) wenigstens ein Bestandteil (2) einer pharmazeutischen und/oder medizinaltechnischen Medikationsvorrichtung (3) aufgenommen ist und die Verpackung (4) in wenigstens einem den Innenraum (5) begrenzenden Abschnitt (6) teildurchlässig ausgebildet ist, so dass ein Dekontaminationsfluid (7), insbesondere Dekontaminationsgas, durchtreten kann und Partikel zurückgehalten werden.

11. Verwendung nach Anspruch 10, **dadurch gekennzeichnet, dass** die Substanz (8) auf einem Diffusionsweg (12) zwischen dem teildurchlässigen Abschnitt (6) und dem wenigstens einen Bestandteil (2) präsentiert wird und/oder dass die Substanz (8) zwischen einer Oberfläche (10) und einer, die Oberfläche (10) nach außen begrenzenden, Schutzschicht präsentiert wird.

12. Verwendung nach Anspruch 10 oder 11, **dadurch gekennzeichnet, dass** die Substanz (8) auf einem gasdurchlässigen und/oder porösen Träger (11), insbesondere Festkörper und/oder textiles Flächengebilde, präsentiert wird.

13. Verwendung nach einem der Ansprüche 10 bis 12, **dadurch gekennzeichnet, dass** das Dekontaminationsfluid (7) außerhalb des Innenraums (5) ausgebracht wird.

14. Verwendung nach einem der Ansprüche 10 bis 13, **dadurch gekennzeichnet, dass** das kontaminationssensible Gebinde (1) nach einem der Ansprüche 1 bis 9 ausgebildet ist.

15. Dekontaminationsvorrichtung (15) mit Mitteln zur Ausführung einer Verwendung nach einem der Ansprüche 10 bis 14, insbesondere innerhalb einer Schleuse (16) für kontaminationssensible, insbesondere pharmazeutische und/oder medizinaltechnische, Gebinde (1), die in eine kontrollierte Umgebung (17) führt.
